Europäisches Patentamt

European Patent Office

Office européen des brevets

(19)

(11) **EP 1 369 680 A1**

(12) # DEMANDE DE BREVET EUROPEEN

(43) Date de publication:
**10.12.2003 Bulletin 2003/50**

(51) Int Cl.⁷: **G01N 21/35**, G01N 30/74,
G01N 21/05, C07C 67/08

(21) Numéro de dépôt: **03291273.5**

(22) Date de dépôt: **28.05.2003**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **03.06.2002 FR 0206781**

(71) Demandeur: **Aventis Pharma S.A.**
**92160 Antony (FR)**

(72) Inventeurs:
• **Nogent, Hélène**
**69730 Genay (FR)**
• **Lagouardat, Jacques**
**93160 Noisy le Grand (FR)**

(74) Mandataire: **Rousseau, Pierrick Edouard et al**
**Aventis Pharma S.A.,**
**20, avenue Raymond Aron**
**92165 Antony Cedex (FR)**

(54) **Méthode de détermination de profils de concentration à partir de profils infrarouges et de données CLHP**

(57) Méthode permettant de définir au cours du temps la concentration des espèces d'un milieu réactionnel à partir des profils infrarouge des produits issus du suivi réactionnel et des données CLHP sans calibration préalable des appareils.

figure 2

Les points correspondent aux données de CHLP et les lignes aux données infrarouge

**Description**

La présente invention concerne une méthode de détermination des profils de concentrations de toutes les espèces présentes dans une même solution sans calibration préalable des appareils de mesure. Généralement sont utilisés un appareil basé sur le principe de la spectrométrie InfraRouge (IR) et toute autre technique d'analyse proportionnelle à la concentration comme par exemple la CLHP (chromatographie liquide haute performance). Cette méthode peut être utilisée pour déterminer la cinétique d'une réaction et déterminer de façon quantitative les différentes entités formées.

Cette méthode permet d'obtenir rapidement un profil quantitatif de toutes les espèces présentes sans étalonnage préalable (IR ou CLHP), d'obtenir les coefficients de réponse UV (ultra violet) de chaque espèce sans qu'elles aient besoin d'être isolées (c'est à dire de façon statistique) et permet de déterminer un modèle reliant l'absorbance IR et la concentration pour chaque produit en tenant compte de l'erreur expérimentale.

Actuellement pour obtenir des résultats quantitatifs à partir des données infrarouge, il est nécessaire de réaliser une calibration de l'infrarouge par acquisition du spectre IR de divers mélanges étalons après déconvolution du signal. En ce qui concerne la CLHP, pour obtenir une vision quantitative de la réaction, il est nécessaire de déterminer les coefficients de réponse UV des composés visibles par un tracé des courbes de réponse UV.

La technique selon l'invention permet de retirer le maximum d'informations de ces deux méthodes d'analyse. Elle est applicable à toute méthode de détermination « en ligne » d'une mesure proportionnelle à la concentration en fonction du temps permettant d'obtenir un grand nombre de données mais dont on ignore le coefficient de proportionnalité par rapport à la concentration.

L'invention a pour objet une méthode statistique permettant d'extraire de deux séries d'analyses qualitatives, des données quantitatives. Dans les études menées précédemment, la demanderesse a constaté que dans la méthode de détermination des profils de concentration à partir des données IR et d'une calibration externe, le bilan matière n'était pas toujours respecté. Cela pouvait être dû à un mauvais étalonnage CLHP ou à des mesures IR biaisées par une absorbance résiduelle non nulle par exemple.

La méthode développée permet d'obtenir un profil de concentration de toutes les espèces sans étalonnage préalable ( IR ou méthode analogue, et HPLC ), d'obtenir les coefficients de réponse UV de chaque espèce sans les isoler et déterminer un modèle reliant l'absorbance IR (ou autre) et la concentration pour chaque produit.

Cette invention a pour objet une méthode de détermination des concentrations des espèces du milieu caractérisée par la mesure de spectres infrarouges en fonction du temps et d'une seconde mesure à partir d'une technique d'analyse proportionnelle à la concentration de l'espèce dans le milieu. La méthode selon l'invention permet donc plus rapidement d'obtenir les données nécessaires à la détermination d'un profil cinétique.

La méthode selon l'invention de détermination des concentrations des espèces du milieu est caractérisée par la détermination dans le milieu des coefficients de proportionnalité infrarouge et CLHP.

La méthode selon l'invention, où la concentration des espèces est déterminée par les profils infrarouges et CLHP, est caractérisée par le fait qu'aucune calibration préalable des appareils infrarouge en ligne et CLHP n'est effectuée.

C'est à dire que les coefficients de proportionnalité du signal infrarouge et du signal CLHP de chaque produit par rapport à la concentration sont inconnus. Seul le nombre de composés identifiés en infrarouge est connus.

L'invention a pour objet l'utilisation d'appareils de spectrométrie infrarouge en ligne, de son logiciel de déconvolution et d'appareil CLHP pour la mise en oeuvre de la méthode d'analyse.

L'invention a aussi pour objet l'utilisation d'appareils de spectrométrie infrarouge en ligne et de son logiciel de déconvolution pour la mise en oeuvre de la méthode d'analyse.

L'invention a aussi pour objet l'utilisation d'appareil de mesure permettant d'obtenir un profil lié à la concentration et au temps pour la mise en oeuvre de la méthode d'analyse.

L'invention a aussi pour objet l'utilisation d'un appareil CLHP pour la mise en oeuvre de la méthode d'analyse.

Cette méthode est illustrée de manière non limitative sur une réaction classique d'estérification en milieu acide de l'acide benzoïque en benzoate d'éthyle. Cette réaction chimique a été suivie par deux techniques d'analyse en ligne: l'infrarouge à transformée de Fourrier (FTIR) et par chromatographie liquide haute performance (CLHP). Ces deux séries d'analyses permettent d'obtenir séparément des analyses « qualitatives » ( évolution d'un produit par rapport à un autre, non évolution, fin de réaction). Du fait du grand nombre de données redondantes issues des 2 séries d'analyse, et après avoir identifié en CLHP les produits détectés en IR, on peut calculer les coefficients de proportionnalité par rapport à la concentration. En combinant ces résultats il a été alors possible d'obtenir les conversions en temps réel, la concentration résiduelle en produit de départ et les coefficients de réponse UV de chaque produit. Le suivi cinétique a pu être réalisé et le modèle cinétique validé.

Cette méthode permet d'exploiter de façon approfondie les deux types d'analyse et d'obtenir plus de renseignements en les combinant que chacune prise séparément c'est à dire de déterminer les coefficients de proportionnalité ( le coefficient de réponse UV statistique pour chaque produit en solution et les paramètres du modèle reliant l'absorbance IR et la concentration de chacune des espèces).

Cette technique nécessite que chacun des composés identifiés en infrarouge soit visible à la fois en CLHP.

Pour utiliser cette méthode, il est nécessaire d'avoir associé chaque produit détecté en infrarouge à un produit détecté en CHLP , il est aussi nécessaire de réaliser un certain nombre de prélèvement CLHP (ou une autre technique d'analyse quantitative ) de façon à avoir les données nécessaires à la résolution mathématique du problème, c'est à dire d'avoir les données nécessaires à la détermination des 4 paramètres : 3 paramètres infrarouge et 1 paramètre CLHP par produit). L'idéal est de réaliser un nombre de prélèvement 2 fois supérieur à celui du nombre de produits observés.

Le principe du programme repose sur la comparaison de tous les signaux enregistrés pour chaque produit et du signal global. Ces réponses sont retravaillées mathématiquement en faisant intervenir les coefficients de réponse et en utilisant un modèle simple de proportionnalité entre l'absorbance mesurée et la concentration tout en respectant les équations du bilan matière à chaque instant t et l'égalité des résultats obtenus pour chaque série d'analyse.

Cette manipulation mathématique permet d'obtenir ainsi à chaque instant t l'égalité des résultats issus des différents systèmes d'analyse pour chacun des produits en chaque instant et l'égalité du signal global.

C'est à dire que pour chaque produit, à chaque instant t, les résultats retravaillés issus de la CLHP et ceux issus de l'infrarouge doivent être identiques et que la somme de ces résultats doit être, elle-aussi, identique. C'est-à-dire pour une réaction où trois produits sont présents dans le milieu :

Concentration produit 1 CLHP (pour chaque prélèvement) = Concentration produit 1 Infrarouge

Concentration produit 2 CLHP (pour chaque prélèvement) = Concentration produit 2 Infrarouge

Concentration produit 3 CLHP (pour chaque prélèvement) = Concentration produit 3 Infrarouge

Somme des concentrations CLHP (pour chaque prélèvement) = Somme des concentrations infrarouge

Somme des concentrations en IR (en tout instant) = bilan matière

La présente invention est illustrée de manière non limitative avec l'exemple suivant où trois produits sont présents dans le milieu.

Cette méthode a été testée sur la réaction d'estérification de l'acide benzoïque en benzoate d'éthyle dans l'éthanol en présence d'acide sulfurique à 50°C. Une sonde IR React IR 1000 est introduite dans le milieu et permet après traitement par déconvolution l' obtention d'un profil d'absorbance en fonction du nombre d'onde et du temps. Selon la loi de Beer-Lambert, les profils infrarouges obtenus sont proportionnel à la concentration dans le milieu. Le profil CLHP est déterminé par des prélèvements réguliers. L'appareil CLHP est composé d'une colonne Inertsil ODS de longueur L=25 cm, de diamètre 4.6 ; de longueur d'onde 220nm, Four 30°C ; éluant 100% Méthanol, Prélèvement 0,1 mL de milieu réactionnel dans 20 mL de Méthanol ; volume injecté 1 µL. Cette méthode permet d'obtenir les coefficients de réponse UV des produits sans avoir à tracer leur droite d'étalonnage CLHP et d'obtenir leurs concentrations à chaque instant dans le milieu.

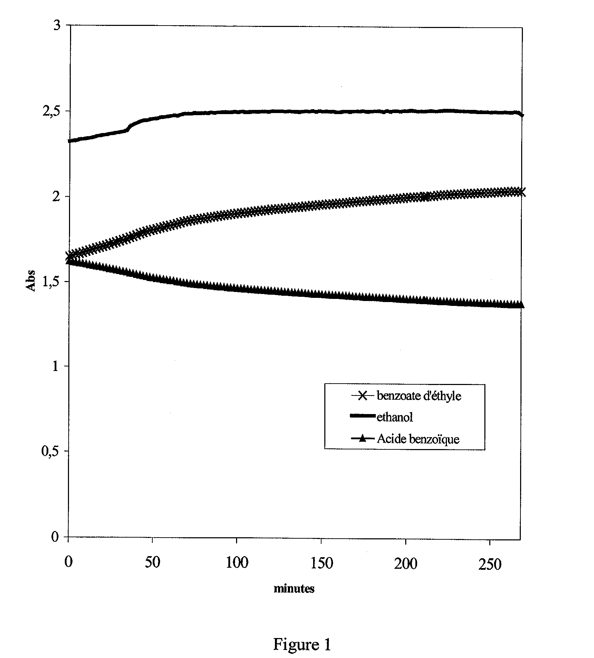

Les données infrarouges utilisées sont représentées sur la figure 1. Il s'agit des profils infrarouges des différents produits obtenus par déconvolution des données brutes infrarouges, ils représentent l'absorbance des produits en fonction du temps.

Les résultats des mesures CLHP sont donnés dans le tableau 1 ci dessous.

Tableau 1

| T (h) | Aire du pic acide benzoïque (ua) | Aire du pic benzoate d'éthyle (ua) |
|-------|----------------------------------|-------------------------------------|
| 0 | 7824759,5 | 86477 |
| 0,233 | 7529081,5 | 218547 |
| 0,666 | 7237722 | 561973 |
| 4,1 | 5685246 | 2018365 |

Par traitement mathématiques de ces profils infrarouges, on obtient les données IR réduites pour les temps de prélèvement de CLHP. Les résultats sont présentés dans le tableau 2

Les données réduites IR sont obtenues par la relation :

Abs réduite = (ABS brute - ABS brute minimale) / (ABS brute maximale -ABS

brute minimale)

où ABS brute minimale est le minimum de la courbe d'absorbance pour le produit considéré et ABS brute maximale est le maximum de la courbe d'absorbance pour le même produit.

Tableau 2

| T (h) | Abs réduite Acide benzoïque | Abs réduite Benzoate d'éthyle |
|---|---|---|
| 0 | 1 | 0 |
| 0,233 | 0,89354125 | 0,10645875 |
| 0,666 | 0,67349637 | 0,32650363 |
| 4,1 | 0 | 1 |
| **ABS brute min** | **1,382** | **1,648** |
| **ABS brute max.** | **1,6204** | **2,041** |

A partir de ces données retravaillées, il est alors possible de calculer les coefficients de réponse UV de l'acide benzoïque et du benzoate d'éthyle(Tableau 3) en choisissant comme modèle simple de proportionnalité :

$$C_i = A_i \times (1/H_i)$$

Dans lequel $C_i$ est la concentration molaire du produit i ; $H_i$ est le coefficient de réponse Aire/concentration molaire calculé et $A_i$ est l'aire du produit i en CHLP.

Tableau 3

| Produit | H statistique | H expérimental |
|---|---|---|
| Acide benzoïque | $4,55.10^6$ | $4,36 \ 10^6$ |
| Benzoate d'éthyle | $4,293.10^6$ | $4,38 10^6$ |

A partir de produits commerciaux les coefficients de réponse mesurés, les valeurs obtenues sont identique à celles trouvées par la méthode statistique décrite ci dessus et reportées dans le tableau 3.
On détermine alors aussi les coefficients du modèle infrarouge reliant l'absorbance et la concentration des espèces.
Le modèle reliant la concentration et l'absorbance est

$$C_{i_t} = \beta io + \beta i_1 * [AbsXri]_t + \beta i_2 *([AbsXri]_t)^2$$

où $C_{i_t}$ est la concentration molaire du produit i dans le milieu à l'instant t
$\beta io$, $\beta i_1$ et $\beta i_2$ les coefficients du modèle de corrélation pour le produit i
$[Abs Xri]_t$ l'absorbance réduite du produit i à l'instant t
Les résultats sont présentés dans le tableau 4

Tableau 4

| Coefficient du modèle | Acide benzoïque | Benzoate d'éthyle |
|---|---|---|
| $\beta$**o** | 1,24897 | 0,0201432 |
| $\beta$**1** | 0,44988 | 0,44987 |
| $\beta$**2** | $-2,6 \ e^{-6}$ | $2,573e^{-6}$ |

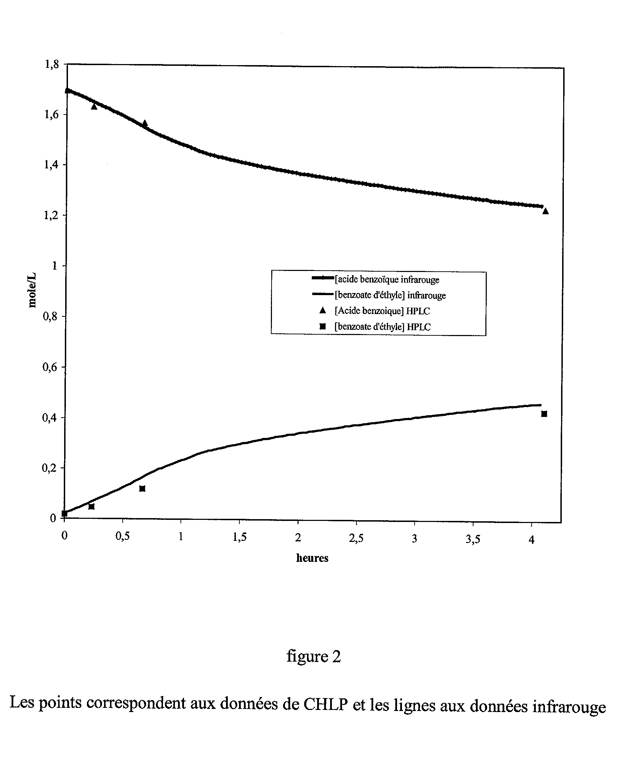

Le profil de concentration obtenu en corrélant les points CHLP et le profils infrarouges est présenté sur la figure 2. Le résidu sur le bilan matière pour l'infrarouge est de 1,3 $10^{-13}$ et le résidu de corrélation entre les résultats CHLP et infrarouges est de 6 $10^{-4}$ . L'égalité des bilans et des résultats est donc bien réalisée. Les profils de concentrations sont donc réalistes.

Cette méthode a permis de retrouver les coefficients de réponse relatifs des produits puis d'obtenir un profil de concentration respectant le bilan de matière à chaque instant et pour chaque série de mesure et correspondant au modèle cinétique connu.

**Revendications**

1. Méthode de détermination des concentrations des espèces du milieu par la mesure de spectres infrarouges en fonction du temps et d'une seconde mesure à partir d'une technique d'analyse proportionnelle à la concentration de l'espèce dans le milieu **caractérisée par** la détermination dans le milieu des coefficients de proportionnalité infrarouge et CLHP.

2. Méthode de détermination des concentrations des espèces du milieu sans calibration préalable des deux appareils par la mesure de spectres infrarouges en fonction du temps avec un appareil à infrarouge et d'une seconde mesure à partir d'une technique d'analyse proportionnelle à la concentration de l'espèce dans le milieu avec un appareil CLHP.

Figure 1

figure 2

Les points correspondent aux données de CHLP et les lignes aux données infrarouge

**Office européen des brevets**

## RAPPORT DE RECHERCHE EUROPEENNE

Numéro de la demande

EP 03 29 1273

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | DD 231 649 A (AKAD WISSENSCHAFTEN DDR) 2 janvier 1986 (1986-01-02) * le document en entier * | 1,2 | G01N21/35 G01N30/74 G01N21/05 C07C67/08 |
| X | WO 02 00760 A (GEN ELECTRIC) 3 janvier 2002 (2002-01-03) * page 2, ligne 1 – ligne 19 * * page 6, ligne 5 – page 7, ligne 14 * * page 8, ligne 1 – page 9, ligne 27 * * page 11, ligne 5 – ligne 15 * * figures 3-8; exemples 6,7 * | 1 | |
| A | WO 95 33705 A (EXXON CHEMICAL PATENTS INC) 14 décembre 1995 (1995-12-14) * page 12, ligne 16 – page 14, ligne 2 * * page 17, ligne 9 – ligne 18 * | 1,2 | |

**DOMAINES TECHNIQUES RECHERCHES (Int.Cl.7)**

G01N
C07C

Le présent rapport a été établi pour toutes les revendications

| Lieu de la recherche | Date d'achèvement de la recherche | Examinateur |
|---|---|---|
| LA HAYE | 2 septembre 2003 | Joyce, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non–écrite
P : document intercalaire

T : théorie ou principe à la base de l'invention
E : document de brevet antérieur, mais publié à la date de dépôt ou après cette date
D : cité dans la demande
L : cité pour d'autres raisons
 
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**  EP 03 29 1273

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

02-09-2003

| Document brevet cité au rapport de recherche | | Date de publication | Membre(s) de la famille de brevet(s) | | Date de publication |
|---|---|---|---|---|---|
| DD 231649 | A | 02-01-1986 | DD | 231649 A1 | 02-01-1986 |
| WO 0200760 | A | 03-01-2002 | US | 6437082 B1 | 20-08-2002 |
| | | | AU | 6165501 A | 08-01-2002 |
| | | | WO | 0200760 A1 | 03-01-2002 |
| WO 9533705 | A | 14-12-1995 | AU | 2816895 A | 04-01-1996 |
| | | | WO | 9533705 A1 | 14-12-1995 |

EPO FORM P0460